# EUROPEAN PATENT APPLICATION

(11) **EP 4 826 726 A1**
(43) Date of publication of application: **21.10.2026**
(21) Application number: 25382384.3
(22) Date of filing: 14.04.2025
(51) Int. Cl.: G16B 15/20, G16B 15/30, G16B 20/50, G16B 40/20, G16B 40/30

(54) **METHOD AND SYTEM FOR DESIGNING ARTIFICIAL ENZYMES**

(71) Applicant: Thenextpangea, S.L., 33418 Gozón - Asturias (ES)
(72) Inventor: Fernández Suárez, Alicia, 33418 Gozón - Asturias (ES); García Osorio, Fernando, 33418 Gozón - Asturias (ES); Soria Valles, Clara, 33418 Gozón - Asturias (ES); Trapote Fernández, Andrea, 33418 Gozón - Asturias (ES)
(74) Representative: Clarke, Modet y Cía., S.L.

(57) **Abstract**

Method (200) and system (100) for designing an artificial enzyme (106). The method (200) comprises: applying (S20) protein language models (110) to an initial enzyme (102) and obtaining sets (103) of substitution probabilities (104) of original amino acids (101) and substitution amino acids (107); selecting (S30) from the sets (103) a candidate group (124) comprising substitution amino acids (107); iteratively, until fulfilling a stopping criterium (133, 147), testing fitness (S50) for a test group (125) belonging to the candidate group (124) and obtaining fitness tested data (131) including a property exhibition information for substitution amino acids (107); training (S60) a machine learning model (142) with fitness tested data (131) and obtaining fitness predicted data (144) including prediction information of a property exhibition for possible substitution amino acids (107) in each position of the initial enzyme (102); selecting a new candidate group (124) further using fitness predicted data (144); generating (S70) an artificial enzyme (106) by replacing in a plurality of positions of the initial enzyme (102), an original amino acid (101) with a substitution amino acid (107) having a prediction value (145) higher than a threshold.

## Description

### TECHNICAL FIELD

The invention described herein relates to the field of methods for designing artificial enzymes. Specifically, the present invention relates to a method for designing new enzymes with improved functions by changing its sequence, through a method. The invention is also related to a system for improving natural enzymes and for designing new enzymes.

### BACKGROUND OF THE INVENTION

Enzymes are proteins with the ability to catalyse biochemical reactions and play an essential role in industry, acting on a wide variety of chemical reactions in a highly efficient and specific manner. Most commercially available enzymes have been modified from their natural sources to alter some of their functional properties, such as temperature, pH or substrate specificity.

Traditional strategies for the evolution of enzyme activities are based on the random introduction of variations in the amino acid sequence of a polypeptide and the subsequent selection of those variants with the most advantageous properties, resulting in enzyme variants with a small number of changes as compared with the natural protein. This is a slow and costly process, which primarily limits the commercial exploitation of these results.

In addition, there is a growing demand for artificial enzymes, which can be defined as the product of a concerted effort to transform a natural enzymatic activity into a variant that has a low degree of sequence similarity but maintains or enhances activity, on the same or over different substrates. The purpose of these artificial enzymes ranges from fundamental research on enzymatic evolution, to obtain enzymes with ability to perform under conditions different from those of the natural counterpart or assuring biosafety in open-field biotechnological applications.

The possibility of using computer simulations or artificial intelligence algorithms has made it possible to drastically accelerate the processes of enzyme evolution. Recent technological advances have greatly facilitated this process by providing the experimental approaches of directed evolution or by enabling computer-assisted applications (Planas-Iglesias, Joan, et al. "Computational design of enzymes for biotechnological applications." Biotechnology Advances 47 (2021): 107696).

Computational techniques can be used to engineer properties such as enzymatic reactivity, substrate specificity, ligand binding, access pathways, ligand transport, protein stability, solubility, flexibility, etc. Theoretical approaches can also identify hotspots on the protein sequence for mutagenesis and predict suitable alternatives for selected positions with expected outcomes (Planas-Iglesias, Joan, et al. "Computational design of enzymes for biotechnological applications." Biotechnology Advances 47 (2021): 107696).

However, current methods of enzyme evolution have important limitations that prevent the creation of scalable pipelines for the improvement of industrial enzymes. These limitations can be divided into two different categories: those related to the source of the input data as well as those related to the enzyme prediction outputs. In this regard, previous methods require experimental or simulated data related to the physical interaction between the enzymes and their substrates. However, our methodology is agnostic on the substrate interaction, which makes it feasible to apply to any kind of known, simulated or predicted proteins.

In terms of output, current methods all focus on the active site of the enzyme and fail to provide sequence or structural alternatives for regions that are not in the vicinity of the active site. In our case the entire protein sequence is optimized, resulting in artificial enzymes with variations throughout the sequence that enhance the functionality of the original enzyme.

For example, US2009191607A1 relates to techniques for computationally designing enzymes. These techniques are used to design variations of naturally occurring enzymes, as well as new enzymes having no natural counterparts. The techniques are based on first identifying functional reactive sites required to promote the desired reaction. Then, hashing algorithms are used to identify potential protein backbone structures (i.e., scaffolds) capable of supporting the required functional sites. These techniques were used to design 32 different protein sequences that exhibited aldol reaction catalytic function. This method can only be used for local enzyme improvement, resulting in variants with few changes from the natural enzyme and limited to the region of the active site where it interacts with the substrate.

US2016063177A1 relates to an automated method of computational enzyme identification and design. Further provided are automated computational design and screening methods to engineer proteins with desired functional activities including, but not limited to ligand binding, catalytic activity, substrate specificity, regioselectivity and/or stereoselectivity. This is a method of predicting the function of a given enzyme. It potentially allows the activity of the enzyme to be improved by replacing one of the amino acids present in the active site. It requires real or predicted data on the 3D structure of the enzyme. It also has the same limitations as the previous method in terms of local improvement (only the active site) and that the result is basically the original enzyme with some specific variation.

EP3891750A1 relates to a method and a computer program for determining a cofactor specificity of a target enzyme, wherein the target enzyme is expected to use one of a first cofactor and a second cofactor based on an amino-acid sequence of the target enzyme, particularly wherein the amino-acid sequence is devoid of 2D- or 3D-structural enzyme information. Another aspect of the invention is to provide a method and computer program for determining an amino-acid sequence of a target enzyme variant, wherein the enzyme variant is characterized by a cofactor specificity differing from that of the target enzyme, wherein the cofactor specificity of the enzyme variant is particularly switched from the first cofactor to the second cofactor.

WO2022233232A1 provides a computer implemented method for designing artificial variants which have activity towards non-natural substrates. The invention provides a special method to process stability evaluation results and creatively combines a process of calculating free energy barrier, which can improve the accuracy of the virtual screening of enzyme variants. The computational method disclosed by this invention greatly reduce the number of variants to be constructed and tested in the wet lab. In some cases, this method achieved the effect of enzyme engineering that cannot be achieved by traditional directed enzyme evolution methods. This method requires detailed knowledge of the interaction between the substrate and the enzyme, which limits the possibility of using this method for a large population or number of enzymes. In addition, thermodynamic data is required for each position to be optimised.

WO2023022783A1 describes a method for producing enzyme mutants includes a generative model parameterized using homologous sequences of wild-type enzymes. Based on a distance between a mutated amino acid residue of each mutant and a corresponding substrate when the substrate is bound to the mutant being less than or equal to a first threshold, the processor may select a first candidate set of enzyme mutants and calculate a statistical energy of each mutant of the first candidate set, and identify, based on the calculated statistical energies, a first set of enzyme mutants among the first candidate set to improve enzyme efficiency. Based on the distance being larger than or equal to a second threshold, the processor may select a second candidate set of enzyme mutants and calculate a statistical energy of each mutant of the second candidate set, and identify, based on the calculated statistical energies, a second set of enzyme mutants among the second candidate set of enzyme mutants to improve protein stability. It is a method for local and therefore highly selective optimisation of some key enzyme positions for interaction with the substrate. It uses 3D and thermodynamic data as input for enzyme improvement.

Therefore, a new method is needed to design artificial enzymes and optimise all parts of the enzyme, not just the active site, without specific knowledge of the nature of the substrate or the mechanism of interaction with the enzyme.

### DESCRIPTION OF THE INVENTION

In light of the above, it is an object of the present invention to address the technical problems present in the state of the art and to provide a method and a system for designing an artificial enzyme with an improved property according to the independent claims. Preferred or advantageous embodiments are also defined in dependent claims. The improved property or properties of the artificial enzyme may be engineered into one or more host cells to enable a certain function.

The approach of the present invention circumvents previous limitations of the techniques used to evolve natural enzymes, being the main feature the ability to operate without specific knowledge of the substrate's nature, or the mechanism of interaction with the enzyme, also the ability to optimize all parts of the enzyme, not only the active site. This makes this invention capable of working with enzymes of any source in a unique and scalable way. The invention iteratively inspects substitution amino acids (i.e., mutations) for at least a fragment of the initial enzyme to find those ones that exhibit a desired property (i.e., activity, stability or substrate specificity) in certain positions of the initial enzyme.

The inventors of the present invention, after extensive and thorough research, have surprisingly discovered the enzyme variants obtained through the proposed method have enhanced properties with respect to their natural variants, for example, higher robustness in terms of temperature or pH, higher enzymatic activity or, in some cases, modified substrate specificities. On the other hand, the proposed method and system represent significant improvements over the state of the art, allowing a highly random process of enzyme improvement to be transformed into a directed flow, and therefore scalable to a variety of different use cases.

Furthermore, it allows previously unexplored solutions to be achieved by identifying variants and, above all, by identifying the best possible combinations of permutations in the amino acid sequence.

The method comprises: applying protein language models to an initial enzyme and obtaining sets of substitution probabilities of original amino acids and substitution amino acids; selecting from the sets a candidate group comprising substitution amino acids; iteratively, until fulfilling a stopping criterium, testing fitness for a test group belonging to the candidate group and obtaining fitness tested data including a property exhibition information for substitution amino acids; training a machine learning model with fitness tested data and obtaining fitness predicted data including prediction information of a property exhibition for possible substitution amino acids in each position of the initial enzyme; selecting a new candidate group further using fitness predicted data; generating an artificial enzyme by replacing in a plurality of positions of the initial enzyme, an original amino acid with a substitution amino acid having a prediction value higher than a threshold.

The system comprises: a model selecting unit configured to apply protein language models to at least a fragment of an initial enzyme and to obtain sets of substitution probabilities, each set associated with a protein language model, so each substitution probability associates an original amino acid in a position of the initial enzyme with a substitution amino acid; a candidate selecting unit configured to select from the sets a candidate group based at least on the substitution probabilities from the protein language models, the candidate group comprising a substitution amino acids, each substitution amino acid corresponding to an original amino acid; a testing unit configured to iteratively test a test group formed by substitution amino acids from a candidate group and to obtain fitness tested data; a fitness prediction unit configured to iteratively train a machine learning model with fitness tested data and to obtain fitness predicted data, the predicted fitness data including prediction information of exhibiting a property for possible substitution amino acids in each position of the initial enzyme, the fitness predicted data being additionally used by the candidate selecting unit to iteratively select and form another candidate group; a generating unit configured to generate an artificial enzyme when the machine learning model reaches a preestablished accuracy and/or, when fitness predicted data values reach a predefined threshold, the artificial enzyme being generated by replacing certain positions of the initial enzyme corresponding to an original amino acid with a substitution amino acid, so that the substitution amino acid has a prediction value higher than a threshold. The system may be implemented in a programable processing device such as a computer.

Preferably, the initial enzyme is selected from the group consisting of enzymes with pectin lyase activity, endoglucanase activity, beta galactosidase activity, exoglucanase activity, alpha-amylase activity, mannan endo-1,4-beta-mannosidase activity, subtilisin activity, lysozyme activity, poly(ethylene terephthalate) hydrolase activity, mono(2-hydroxyethyl) terephthalate hydrolase activity, cutinase activity, DNA polymerase activity, reverse transcriptase activity and deoxyribonuclease activity.

In an embodiment of the present invention, a non-transitory computer readable medium is provided. The medium stores executable instructions that when executed by a computer performs at least the steps of the independent method claim.

The present invention may use a recombinant system of heterologous species selected from a bacteria, yeast, fungi or insect.

The enzyme designed by the method and system of the present invention shows improvements in its properties such as activity, stability, broader or altered substrate specificity. In an embodiment

The terms "initial sequence of an enzyme" and "initial enzyme" are used interchangeably in the present invention.

### BRIEF DESCRIPTION OF DRAWINGS

The following figures are described below. These illustrate exemplary and non-limiting embodiments for a better understanding of the invention.
FIG. 1A is a workflow diagram according to an embodiment of the invention.
FIG. 1B is a block diagram according to an embodiment of the invention.
FIG. 2 is an example of the output of one Protein Language Models (PLM).
FIGs. 3A-3C illustrate three combination criteria.
FIG. 4 is a diagram of the iterative loop involving the generation, testing, and prediction of the substitutions.

### DETAILED DESCRIPTION

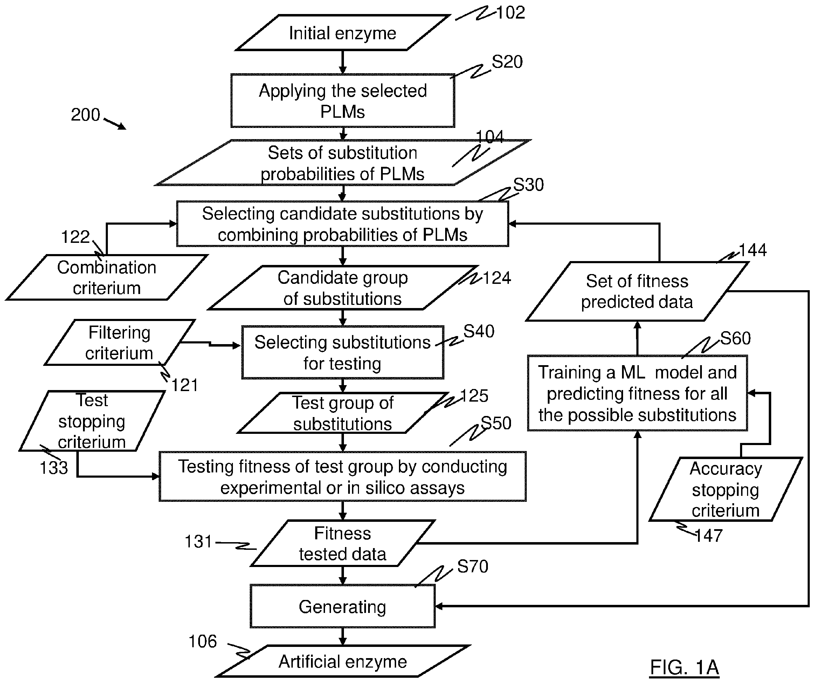

FIG. **1A** depicts a simplified flowchart of an embodiment of a method 200 for designing an artificial enzyme 106. In some embodiments, the whole method 200 can be performed in a processing device (e.g., a computer). The method 200 encompasses several steps and uses an initial enzyme 102 (or a fragment of an initial enzyme).

A step S20 for applying several protein language models (also referred to as PLM) to the initial enzyme 102 is carried out. A PLM is a type of self-supervised deep learning model specifically adapted to protein sequences and structures. These PLMs are trained to understand and predict protein properties based on contextualized embeddings derived from amino acid sequences. By leveraging high-dimensional vector representations, PLMs capture both local residue-level interactions and global structural relationships, enabling a more comprehensive understanding of sequence-function relationships. Preferably, the selected PLMs are the latest versions of existing models, trained on diverse databases containing millions of protein sequences and leveraging cutting-edge neural network architectures, such as Transformers. The step S20 returns substitution probabilities 104 for all positions in the initial sequence 102, and all existing amino acids, generated by each of the protein language models. It is noted that a substitution probability associates a value for substituting an original amino acid in the initial enzyme 102 with another amino acid, namely the substitution amino acid.

The method 200 continues in a step S30 for selecting candidate substitutions from the PLMs following a certain combination criterium 122, as a result a candidate group 124 of unique substitutions for each position are obtained.

The method 200 continues in a step S40 for selecting substitutions for certain positions for testing while excluding others, so as to obtain a test group 125 and to proceed to step S50. Those amino acid substitutions of the test group 125 are used in a step S50 for testing in order to determine their fitness by conducting experimental or in silico assays. The specific definition of fitness depends on the use case.

The step S50 produces fitness tested data 131 to be used in step S60 for trainining a machine learning model (ML model), like a regressor, and obtaining fitness predicted data 144 with predicted fitness for all possible substitutions. The fitness predicted data 144 is used in the selecting candidate substitutions step S30 to generate a new selection of substitutions for each position 124.

The method 200 may continue with further steps in a feedback loop. When a desired fitness is reached, a new artificial enzyme 106 is generated. The loop stops when a test stopping criterium 133 is fulfilled and/or an accuracy stopping criterium 147 associated to the step S60 is fulfilled (typically, the ML model does not substantially improve results).

The previous steps S20-S60 can be coded as executable instructions for execution by a processing device. Consequently, the testing step S50 may determine fitness of selected substitutions, by conducting in silico assays (using a processing device). Yet, in some embodiments, the testing step can alternatively or additionally include conducting experimental assays.

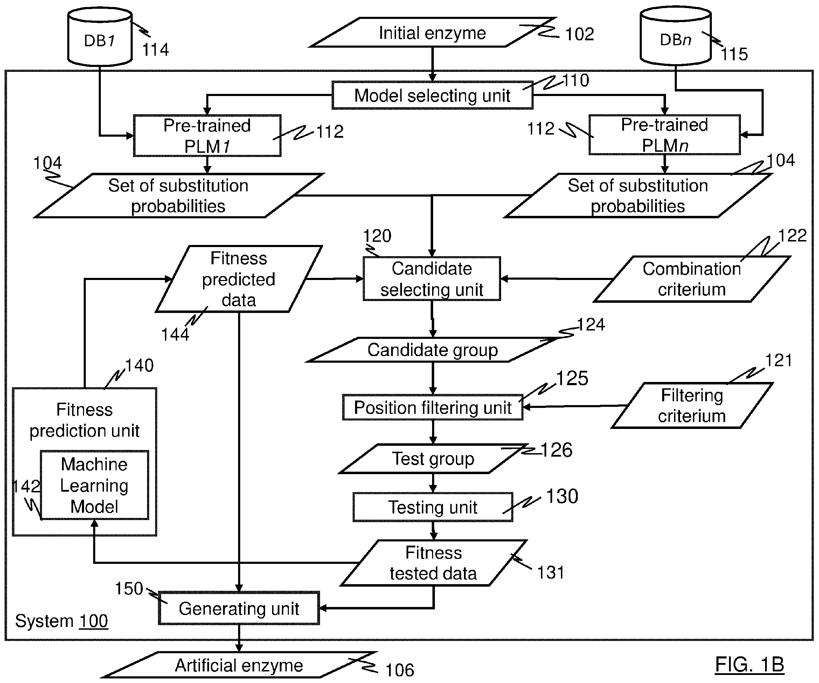

In **FIG. 1B** a block diagram is presented to show how components work and cooperate in an embodiment of a system 100 for designing artificial enzymes 106. The whole system 100 or part of it may be deployed as a processing device, such as a computer.

Firstly, a model selecting unit 110 receives an initial enzyme (or a fragment of an enzyme) 102 as input data and selects among several pre-trained models based on a particular type of natural language processing (NLP) techniques, known as Protein Language Models (PLMs) 112.

Each selected PLM 112 has been trained using one or more corresponding protein sequence databases 114, 115, such as UniRef50, UniRef90, UniProt, and Swissprot, and they potentially encapsulate distinct protein sequence interpretations to offer a more holistic view when integrated. For instance, some protein sequence databases 114, 115 compile protein sequences that exhibit a controlled degree of sequence similarity -no two sequences share more than 50% (UniRef50) or 90% (UniRef90) similarity- to eliminate biological redundancy. Representative examples of selectable PLMs 112, 113 include ESM-1v, ESM-MSA-1b, ESM2, AA-0, AMPLIFY and BERT. However, other existing models can be used, or even new models can be built (e.g., specifically trained) to fit particular needs.

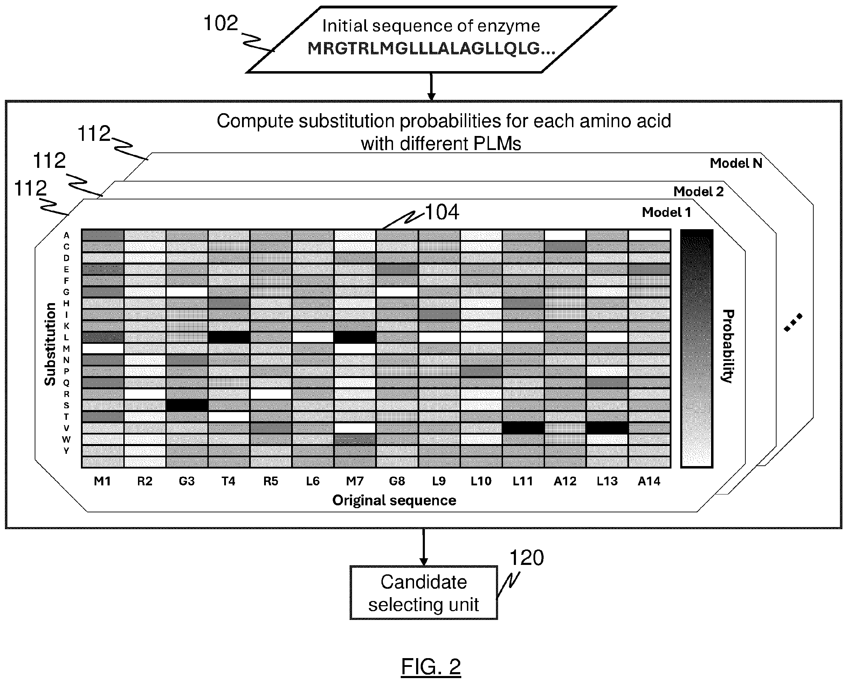

Each PLM 112 generates a set 104 of substitution probabilities for all amino acids at all positions in the initial enzyme 102, indicating the likelihood of substitution to any other existing amino acid. An example of the output of a PLM is shown in FIG. 2.

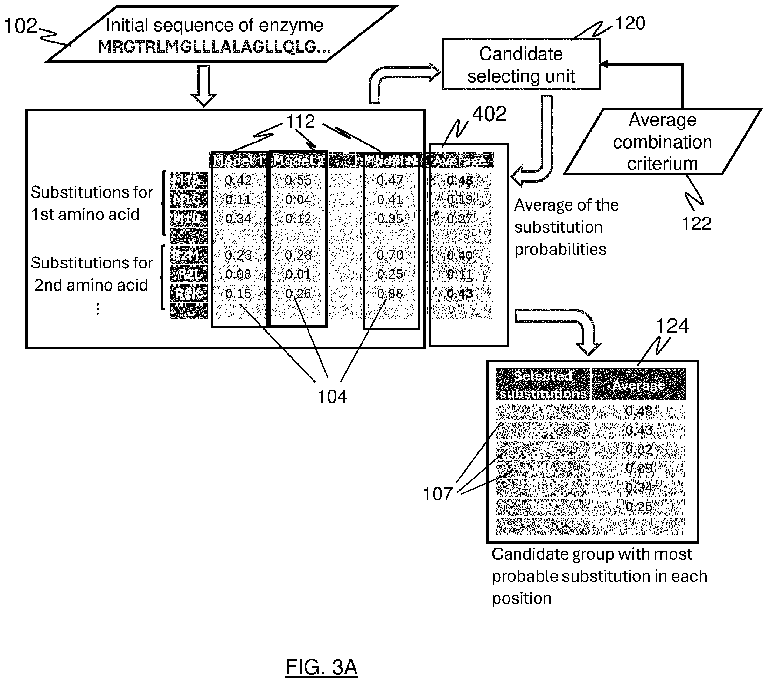

A candidate selecting unit 120 receives sets of substitution probabilities 104 obtained from each PLM 112 and applies a combination criterium 122 to generate a candidate group 124 with selected substitutions, choosing one substitution for each amino acid at every position in the initial enzyme 102. Possible combination criteria 122 are mentioned in FIGs. 3A-3C. For instance, the combination criterium 122 can be defined to award higher scores to substitutions consistently predicted across multiple models, reinforcing the predictive confidence.

A portion of the selected substitutions are used by a position filtering unit 125 to produce a test group 126 of substitutions to be validated individually in a testing unit 130. The decision is based on a filtering criterium 121, for example, prioritizing substitutions that show the highest potential for increased activity or stability -termed 'fitness', or substitutions with high probability but in separated positions across the sequence. The testing unit 130 validates the substitutions 126 of certain positions of the original enzyme 102 by conducting in silico or experimental assays to collect fitness tested data 131, leveraging iterative feedback to continuously enhance prediction accuracy of a fitness prediction unit 140.

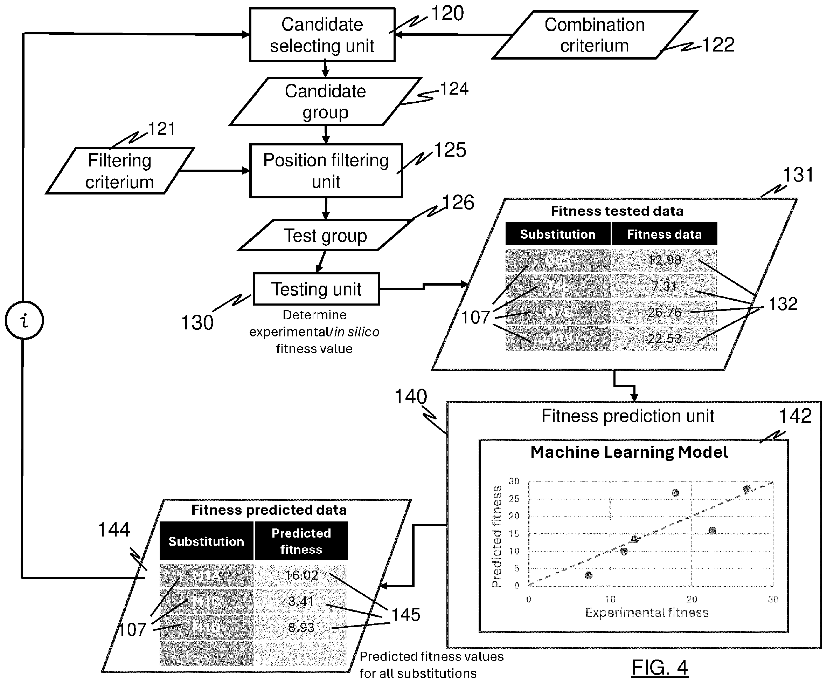

A Machine Learning model 142 (ML model) included in the fitness prediction unit 140, like a regression model or a generative model, is (re)trained using the fitness tested data 131, with input features derived from the PLM models results. The ML model 142 is used in the fitness prediction unit 140 to predict the fitness of each possible substitution 104 of the initial enzyme 102, obtaining fitness predicted data 144 that guide the selection of substitution candidates for further validation in the candidate selection unit 120. Through subsequent iterations (e.g., rounds), the fitness tested data 131 re-trains and refines the ML model 142, optimizing its performance. FIG. 4 represents a detailed view of the candidate selecting unit 120 - position filtering unit 125 - testing unit 130 - fitness prediction unit 140 loop.

Depending on the specific needs, the most suitable ML model 142 is chosen to be part of the fitness prediction unit 140, as well as the number of validated substitutions in each iteration, demonstrating the adaptability and scalability of this approach. The number of iterations is normally determined by the degree of fitness improvement achieved by the substitutions in each iteration and the desired fitness value, which may vary depending on the specific use case.

The artificial enzyme 106 is devised and/or created in a generating unit 150 and it may contain a single substitution or multiple ones, depending on the potential of the obtained substitutions or the objectives for each use case. In some embodiments, the degree of aimed dissimilarity dictates the number of substitutions applied to the artificial enzymes 106. An in silico validation is performed by the generating unit 150, ensuring that the proposed artificial enzyme 106 folds correctly and maintain its three-dimensional structure. The validation is carried out using advanced Al models like AlphaFold, FoldSeek, or RoseTTaFold. This validation is crucial to verify that the functional integrity of the enzyme is preserved, paving the way for the development of new enzymes with enhanced features.

**FIG. 2** illustrates a portion of a heat map as an output of a specific PLM 112 applied to the initial sequence 102 of an example case for artificial enzyme variants with Deoxyribonuclease type 1 activity. The original sequence 102 belongs to the species *Bos taurus.*

The heat map shows the substitution probability 104 of amino acids along the horizontal X-axis (original amino acids) against the vertical Y-axis (possible substitution amino acids), with a grey scale denoting the probability value. The notation is "initial amino acid - position - substitution amino acid'. For instance, several black cells can be selected having a high probability value, this would provide a set of selected substitutions: M1L, G3S, T4L, M7L, L11V, and L13V for this specific PLM.

The substitution probabilities 104 of the selected PLMs 112 are the inputs to the candidate selecting unit 120, where one substitution is selected for each position. Three different strategies for selecting substitutions are explained, as examples of possible combination criterium in FIGs. 3A -3C.

**FIG. 3A** illustrates the application of an average combination criterium 122 for selecting substitutions in the enzyme's initial sequence 102. A candidate selecting unit 120 receives several substitution probabilities 104 from several PLMs 104 (in columns) for each amino acid possible substitution 107 (in rows), and computes, for each row, an average probability value 402. The notation in the tables is 'Initial amino acid - position - substitution amino acid'. Then, the candidate selecting unit 120 produces a candidate group 124 with a selected substitution for each amino acid position using the average combination criterium 122 that is based on the maximum average probability of occurrence among the selected PLMs. For example, substitution T4L has the higher average probability with a value of 0.89, followed by substitution G3S with an average probability value of 0.82.

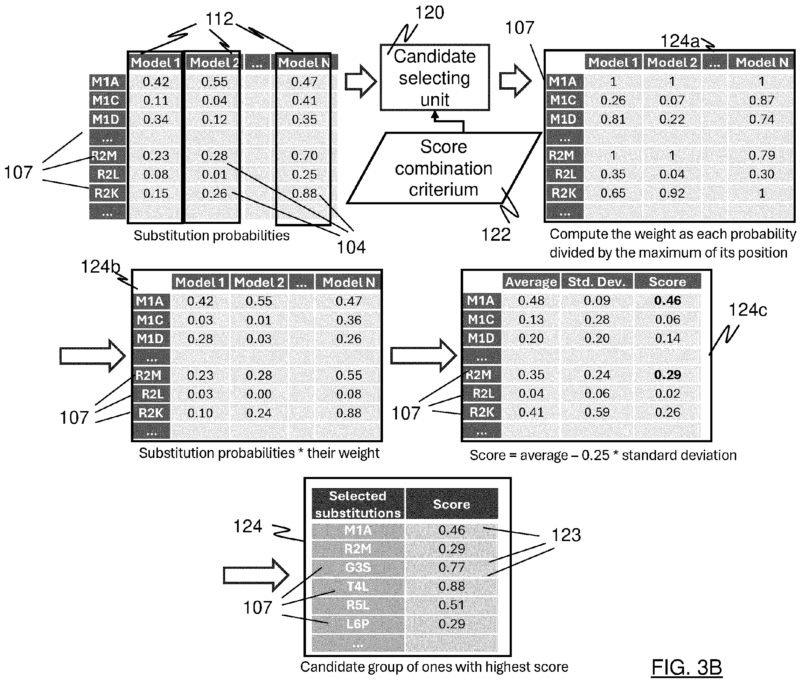

**FIG. 3B** summarizes another combination criterium 122: the score. The candidate selecting unit 120 receives several substitution probabilities 104 from several PLMs 112 (in columns) for each amino acid possible substitution (in rows). Initially, a first intermediate table 124a is obtained by assigning a weight to each substitution 107 at every position by dividing the substitution's probability by the maximum probability at that position, independently for each PLM 112. This approach ensures that the substitution 107 with the highest probability 104 at a given position is assigned a weight of 1, while all other substitutions 107 at that position receive proportionally lower weights, reflecting their lower likelihood of occurrence. Next, a second intermediate table 124b is obtained as follows: the probability of each substitution 107 is adjusted by multiplying it by its corresponding weight, resulting in a weighted probability. This weighting process is applied independently for the selected PLMs 112, yielding weighted probabilities for each PLM 112. A score 123 for each substitution 107 is then calculated by incorporating both the mean and the standard deviation of the weighted probabilities across all selected PLMs, this way a third intermediate table 124c is produced. Substitutions 107 that exhibit strong consensus among the models 112 (indicated by a lower standard deviation) are given higher emphasis with this criterium. The scoring formula is defined in this particular example as: *Score*=*Mean-0.25×Standard Deviation.* Finally, at each position, the substitution 107 with the highest score 123 is identified as the optimal candidate, resulting in the generation of a candidate group 124 with the selected substitutions 107.

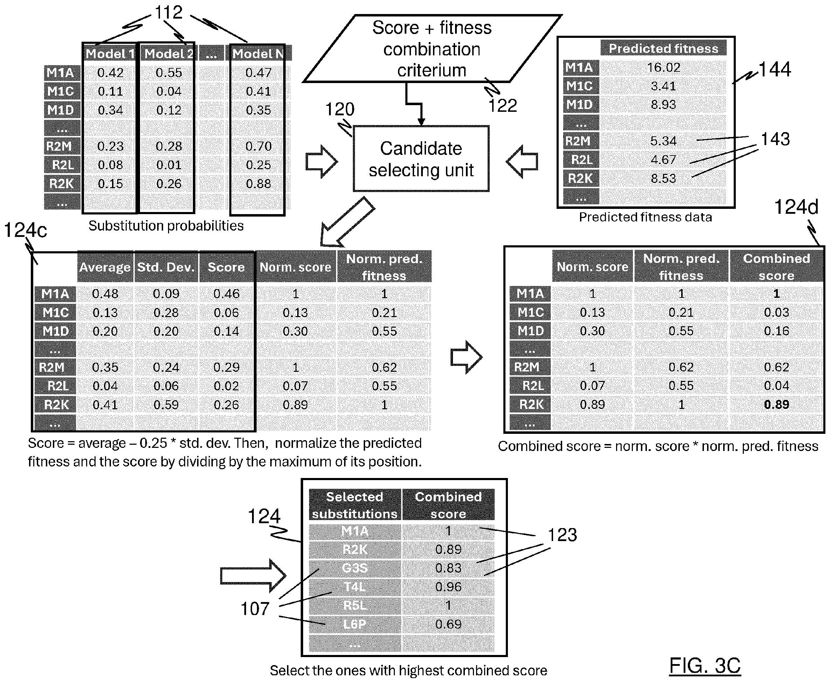

**FIG. 3C** illustrates another combination criterium 122, named score and fitness criterium, where, for each possible substitution, in addition to the substitution probabilities 104 from the PLMs 112, the fitness predicted data 144 from a Machine Learning model 142 are also considered. By combining the prediction values 143 and substitution probabilities 104 a candidate group 124 is formed, the selection of substitutions 107 becomes more robust and offers the advantage of guiding the selection toward improving one or more specific properties-namely, the fitness. According to this approach, firstly the score 124c is calculated as described in FIG. 3B. This score is then normalized by dividing it by the maximum value at the respective position. The predicted fitness is similarly normalized so that both values range between 0 and 1. The combination 124d is computed by multiplying the normalized score by the normalized predicted fitness, balancing likely substitutions with high-fitness ones, and penalizing those with low probability or low fitness. At each position, the substitution with the highest combined score is selected, resulting in a candidate group 124 of selected substitutions 107.

**FIG. 4** explains an iterative loop involving the candidate selecting unit 120, position filtering unit 125, testing unit 130, the ML model 142, and fitness prediction unit 140, previously introduced in FIGs. 1A-1B.

Firstly, the candidate group 124 with the selected substitutions for each position in the initial sequence produced by the candidate selecting unit 120 are processed by the position filtering unit 125. These selected substitutions of the candidate group 124 are filtered by the position filtering unit 125 to produce a test group 126 with a subset of substitutions 107, according to a certain filtering criterium 121, such as random criterium, or the highest score criterium, depending on the use case.

The test group 126 is used for experimental or *in silico* evaluation of the property or properties targeted for improvement in the enzyme, such as activity or stability, in the testing unit 130. These properties are collectively referred to as "fitness" as mentioned before. Similarly, the number of substitutions 107 taken for testing to form the test group 126 may vary depending on the use case, as the initial sequences can have diverse lengths and catalytic centers. In the example shown, four substitutions are evaluated: G3S, T4L, M7L, and L11V.

Next, the fitness tested data 131 obtained from these substitutions 107 serve as input for the ML model 142, which is (re)trained to establish a relationship between features extracted from different results of the PLMs and the test values 132. This trained ML model 142 is then used to predict the fitness of all possible substitutions 107 in the fitness prediction unit 140.

The resulting fitness predicted data 144 is fed into the candidate selecting unit 120, where the combination criterium 122 is applied, to refine the selection of substitutions for each position of the candidate group 124. The fitness predicted data 144 includes a prediction value 145 for each possible substitution 107 of each original amino acid in the initial enzyme. An example of how the fitness predicted data 144 is used to help guide the selection, in combination with the PLMs probabilities 104, is shown in FIG. 3C.

For each specific use case, two parameters are predefined: the desired level of accuracy for the ML model 142 and the acceptable limits for the fitness values. Accuracy is obtained by comparing the predicted fitness values with the measured fitness. The number of iterations (i) depends on the specific use case via stopping criteria that includes a test stopping criterium 133 and an accuracy stopping criterium 147. For each use case, the iterative loop stops when the ML model 142 achieves the desired accuracy, the fitness predicted data 144 values reach predefined acceptable limits or a combination of both are met.

For instance, for a specific protein the two parameters are predefined. The desired ML model accuracy is 90% and the test value 132 greater than 12 units. For this use case, the iterative loop stops when the model either reaches the desired accuracy (90%) or the fitness tested data 131 includes a test value 132 for at least a substitution 107 that is higher than 12 units. These units are an indication of a degree of exhibiting a certain property (i.e., activity or stability).

It will be clear to a skilled person that the invention is not limited to the particular embodiments described above, but the invention will include all embodiments falling within the scope of the appended claims.

## Claims

1. Method (200) for designing an artificial enzyme (106), **characterized in that** the method (200) comprises the following steps:
- applying (S20) a plurality of protein language models (110) to at least a fragment of an initial enzyme (102) and obtaining a plurality of sets (103) of substitution probabilities (104), wherein each set (103) is associated with a protein language model (110), wherein each substitution probability (104) associates an original amino acid (101) in a position of the initial enzyme (102) with a substitution amino acid (107);
- selecting (S30) from the sets (103) a candidate group (124) based on the substitution probabilities (104) from the protein language models (110), the candidate group (124) comprising a plurality of substitution amino acids (107), each substitution amino acid (107) corresponding to an original amino acid (101);
- performing an iterative loop, wherein each iteration of the loop comprises:
- testing fitness (S50) for a test group (125) and obtaining fitness tested data (131), wherein the test group (125) comprises a plurality of substitution amino acids (107) selected from the candidate group (124), wherein the fitness tested data (131) includes a test value (132) indicative of exhibiting a property for each substitution amino acid (107) of the test group (125);
- training (S60) a machine learning model (142) with fitness tested data (131) and obtaining fitness predicted data (144), wherein the predicted fitness data (144) includes a prediction value (145) indicative of exhibiting a property for each possible substitution amino acid (107) in each position of the initial enzyme (102);
- selecting a new candidate group (124) comprising a plurality of substitution amino acids (107) for each original amino acid (101) based on the substitution probabilities (104) from the protein language models (110) and the fitness predicted data (144) from the machine learning model (142);
- stopping the iterative loop when either fitness predicted data (144) or fitness tested data (131) fulfils a corresponding stopping criterium (133, 147);
- generating (S70) an artificial enzyme (106) by replacing in a plurality of positions of the initial enzyme (102), an original amino acid (101) with a substitution amino acid (107) from at least one iteration, the substitution amino acid (107) having a prediction value (145) higher than a threshold.

2. The method of claim 1, wherein it further comprises pre-training the protein language models (112) with a protein sequence database (114) exhibiting a known degree of sequence similarity.

3. The method of claim 1 or 2, wherein the test group (125) for testing fitness (S50) is formed according to a filtering criterium (121) based at least on information about substitution amino acids (107) exhibiting a property.

4. The method of any of claims 1 to 3, wherein the stopping criterium is an accuracy stropping criterium (147) based on whether the machine learning model (142) reaches a preestablished accuracy and/or, wherein the stopping criterium is a test stopping criterium (133) based on whether a plurality of values in the fitness predicted data (144) reach a predefined threshold.

5. The method of claim 4, wherein the machine learning model (142) is a regression learning model or a generative learning model.

6. The method of any of claims 1 to 4, wherein selecting the candidate group (124) follows a combination criterium (122) based on a maximum average of substitution probabilities (104) and/or on a lower standard deviation of substitution probabilities (104).

7. The method of any of claims 1 to 6, wherein the step of testing fitness (S50) for a test group (125) comprises conducting an *in silico* assay.

8. The method of any of claims 1 to 7, wherein the step of testing fitness (S50) for a test group (125) comprises conducting an experimental assay.

9. The method of any of claims 1 to 8, further comprises a step of expressing the sequence of the artificial enzyme (106) in a recombinant system of heterologous species selected from a bacteria, yeast, fungi or insect.

10. System (100) for designing an artificial enzyme (106), **characterized in that** the system (100) comprises:
- a model selecting unit (110) configured to apply a plurality of protein language models (110) to at least a fragment of an initial enzyme (102) and to obtain a plurality of sets (103) of substitution probabilities (104), wherein each set (103) is associated with a protein language model (110), wherein each substitution probability (104) associates an original amino acid (101) in a position of the initial enzyme (102) with a substitution amino acid (107);
- a candidate selecting unit (120) configured to select from the sets (103) a candidate group (124) based at least on the substitution probabilities (104) from the protein language models (110), the candidate group (124) comprising a plurality of substitution amino acids (107), each substitution amino acid (107) corresponding to an original amino acid (101);
- a testing unit (130) configured to iteratively test a test group (126) and to obtain fitness tested data (131), wherein the test group (125) comprises a plurality of substitution amino acids (107) selected from the candidate group (124), wherein the fitness tested data (131) includes a test value (132) indicative of exhibiting a property for each substitution amino acid (107) of the test group (125);
- a fitness prediction unit (140) configured to iteratively train a machine learning model (142) with fitness tested data (131) and to obtain fitness predicted data (144), wherein the predicted fitness data (144) includes a prediction value (145) indicative of exhibiting a property for each possible substitution amino acid (107) in each position of the initial enzyme (102), wherein the fitness predicted data (144) additionally used by the candidate selecting unit (120) to iteratively select a new candidate group (124);
- a generating unit (150) configured to generate an artificial enzyme (106) when the machine learning model (142) reaches a preestablished accuracy and/or, when a plurality of values in the fitness predicted data (144) reach a predefined threshold, wherein the artificial enzyme (106) is generated by replacing in a plurality of positions of the initial enzyme (102), an original amino acid (101) with a substitution amino acid (107) from at least one iteration, the substitution amino acid (107) having a prediction value (145) higher than a threshold.

11. The system (100) of claim 10, wherein the model selecting unit (110) is configured to select the protein language models (112) based on a degree of sequence similarity associated therewith.

12. The system (100) of claim 10 or 11, wherein the machine learning model (142) is a regression learning model or a generative learning model.

13. The system (100) of any of claims 10 to 12, wherein the testing unit (130) is further configured to conduct an *in silico* assay for the test group (125).

14. A non-transitory computer readable medium comprising executable instructions, wherein the instructions, when executed by a processing device, perform at least the steps of any of claims 1 to 7.
